# EUROPEAN PATENT SPECIFICATION

(11) **EP 2 266 949 B1**
(45) Date of publication and mention of the grant of the patent: **29.05.2013**
(21) Application number: 09425226.9
(22) Date of filing: 10.06.2009
(51) Int. Cl.: C07C 231/00, C07C 233/25

(54) **"Fast, high-efficiency quantitative synthesis of paracetamol"**
Schnelle, hocheffiziente, quantitative Synthese von Paracetamol
Synthèse quantitative haute efficacité rapide de paracétamol

(43) Date of publication of application: 29.12.2010
(73) Proprietor: Gregorio, Liberata, 84125 Salerno (IT)
(72) Inventor: Venneri, Filippo, 84125 Salerno (IT)
(74) Representative: Cioncoloni, Giuliana

(56) References cited:
- EP-A- 0 435 263
- US-A- 4 080 505
- US-A1- 2004 138 509
- "Ullmann's Encyclopedia of Industrial Chemistry, volume 2, 6th ed." 2003, WILEY-VCH , WEINHEIM , XP002551387 Page 530: 4-Hydroxyacetanilide.
- DATABASE BEILSTEIN [Online] BEILSTEIN INSTITUTE FOR ORGANIC CHEMISTRY, FRANKFURT-MAIN, DE; XP002551396 Database accession no. 2240154 (BRN) & LIEBIGS ANNALEN DER CHEMIE, vol. 6, 1990, pages 509-512,
- DATABASE BEILSTEIN [Online] BEILSTEIN INSTITUTE FOR ORGANIC CHEMISTRY, FRANKFURT-MAIN, DE; XP002551397 Database accession no. 2329887, 2356843 (BRNs) & ARZNEIMITTEL FORSCHUNG, vol. 30, no. 5, 1980, pages 751-758,

## Description

### TECHNICAL FIELD

The present invention relates to a method for the production of paracetamol.

### PRIOR ART

Paracetamol is an active metabolite of phenacetin resulting from the oxidizing reaction of O-dealkylation.

Processes for the preparation of paracetamol are described for instance in US 2004/0138509 A and EP-A-0 435 263.

Traditionally paracetamol is produced by nitration of phenol with formation of para-nitrophenol 1 and subsequent reduction and acetylation of this one with formation of paracetamol, according to the following reaction scheme:

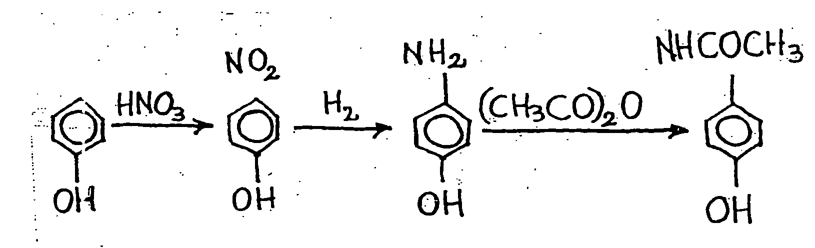

However, the traditional method for producing paracetamol is affected by two basic shortcomings:
(1st) the reactions that take place in it are not quantitative with respect to paracetamol, owing to the fact that in the nitration of phenol - to obtain the para-nitrophenol of interest, this one being the precursor of paracetamol - however the reaction product in the position orto- of phenyl ring, is equal to as much as 66%, both because one has two equivalent attachment positions in the position orto- and only one in the position para-, and for the formation of a hydrogen bond between hydroxyl and one of the two oxygen atoms of nitro group (-NO₂), which bring a partial negative charge and therefore in this case form a very strong hydrogen bond, energetically favouring orto-nitrophenol with respect to para-nitrophenol of interest;
(2nd) it results in byproducts which can hardly be separated and are polluting.

### DISCLOSURE OF THE INVENTION

In view of the shortcomings referred above, it is the object of the present invention to provide a method for a fast, high-efficiency quantitative synthesis for the production of paracetamol.

It is a further object of the present invention to provide a synthesis method for the production of paracetamol, that is also not polluting.

Such objects are achieved according to the present invention with a method for the production of paracetamol including the stages of:
(a) reacting phenol and neopentyl bromide or sodium phenoxide and neopentyl iodide in a solvent, with formation of neopentylphenyl ether;
(*b*) nitrating said neopentylphenyl ether with formation of a nitro compound of neopentylphenyl ether with an NO₂-group in position para- ;
   the nitration gives a strong prevalence of reaction product in the position para-, so that all subsequent stages are quantitative and fast reactions, without polluting or hardly separable byproducts such as in the acetylation which takes place in the traditional synthesis of paracetamol, which is not quantitative and brings to many products to be separated;
*(c)* catalytically reducing said nitro compound with hydrogen with formation of a phenyl amine;
*(d)* acetylating said phenyl amine in position para-;
the acetylation takes place quantitatively and very fastly, because an unstable intermediate reaction product is formed, i.e. acetylpyridinium chloride, which is even more reactive than acetyl chloride and reacts fastly with amino group, acetylating it in a quantitative manner, achieving the objects of the present invention.

Moreover, acetyl chloride costs much less than acetic anhydride and having a molecular weight lesser than acetic anhydride, the weight being equal one has more moles of acetyl chloride and then the economic advantage increases, achieving the objects of the present invention.

A disadvantage of the traditional synthesis of paracetamol is that in order to avoid the acetylation of oxygen, in this case one having to acetylate a para-amino phenol, one is to use acetic anhydride, which is much less reactive than acetyl pyridinium chloride; the reaction is to be carried out under particular conditions in order to avoid the acetylation of the hydroxyl and therefore the product is not quantitative. Moreover, though not willing to be bound to any theory, the Inventor holds that as oxygen is an attractor of electrons, it is the amino group, which is lesse electronegative, that tends to have the conjugative effect with the benzene ring of para-amino phenol. For this reason the nucleophility is depressed, and moreover in the resonance form, in which nitrogen conjugates the doublet with the benzene ring, one has a negative charge of 33% in position para- in which oxygen is bound. Therefore, oxygen becomes richer of electrons and therefore more nucleophilic. The Inventor holds therefore that hydroxyl is acetilated too in a minimum portion, so a lot of products resulting, to be separated: non-acetylated para-amino phenol, the paracetamol of interest, para-amino phenol acetate and acetic acid, which is the byproduct of acetic anhydride. As opposite, in the inventive method one only has insoluble pyridinium chloride as the byproduct of the fourth reaction in the environment, and the pure product soluble in pyridine.

Pyridine chloride can be titrated with soda and thereafter, once the sodium chloride removed, the pyridine can be recovered by azeotropic distillation. In this way there is a recovery of the solvent and one has no environmental effect in wasting the residues of the synthesis, these ones being water and sodium chloride, achieving the inventive objects;
*(e)* cleaving said neopentylphenyl ether with hydrobromic acid in an inert solvent or respectively with hydriodic acid in an inert solvent, with formation of paracetamol and neopentyl bromide or respectively neopentyl iodide.

Advantageously, the inventive method further includes the stage of:
*(f)* recovering said paracetamol by filtration, and recovering said neopentyl bromide or respectively neopentyl iodide by distillation and returning it to stage *(a).*

By virtue of returning neopentyl bromide or neopentyl iodide to the first reaction, though the inventive method envisages two additional stages with respect to the traditional method, however one does not have an economic loss as regards the initial reactant, this one being recovered pure in the final reaction.

The synthesis of paracetamol taught by the present invention turns out to be of a high efficiency, and advantagoeus from the economical standpoint, because the only additional reactant that is employed is hydrobromic acid or hydriodic acid, which is a low-cost product.

The advantages of the inventive method, definitely, derive from the fact that whilst in traditional synthesis attachments are predominatingly formed in position orto-, in the inventive synthesis attachments in position para- are predominatingly obtained.

Therefore, it is the subject of the present invention a method for producing paracetamol according to annexed Claim 1.

It is also the subject of the present invention a method for producing paracetamol according to annexed Claim 2.

### BEST WAY FOR CARRYING OUT THE INVENTION

The present invention will be fully understood based on the following detailed description of a preferred embodiment thereof, only given as a matter of example, absolutely not of restriction of the teaching provided thereby.

The inventive method for producing paracetamol includes five reactions.

The first reaction is a reaction between phenol and neopentyl bromide in triethylamine, with formation of neopentylphenyl ether and triethyl ammonium bromide. In the first reaction also hydrobromic acid is formed. The solvent (triethylamine), protonated by hydrobromic acid, may be recovered by titrating it with soda, which forms sodium bromide and water, and by simply separating it after filtering sodium bromide, as water and triethylamine are not miscible with each other, and in case one would proceed by distillation, they do not form an azeotrope and therefore the distillation is simple to be brought to its end, and so the triethylamine re-enters the production cycle and waste products are not polluting, being water and sodium bromide, so the inventive objects being achieved.

It is envisaged that the phenol may also be under the form of the derivative of phenol composed of sodium phenoxide, reacted with neopentyl iodide in a suitable solvent.

The second reaction is a nitration of neopentyl phenyl ether formed in the first reaction. The nitration can be carried out with the known nitrating mix, composed of nitric acid and sulfuric acid in an inert solvent, such as e.g. tetrahydrofuran or the cheaper like.

Oxygen in the ethereal form always keeps a conjugative effect with benzene ring which is orto- and para- orientating. Rather, the union of an electronreleasing alkyl group increases the conjugative effect, whereby the reaction proceeds in an even more efficient way. The product of this second reaction is predominatingly in position para-. Without willing to be bound to any theory, it is held that this ensues from two factors: (1st) the high directive power of neopentyl group, owing to the high steric encumbrance thereof. During the reaction, it makes the attachment more difficult of nitronium cation on positions orto-; (2nd) a repulsive effect between alkyl groups donors of electrons and the two partially negative oxygen atoms present on nitrogen when the product in position orto- is formed, which moreover restricts the rotational degrees of freedom and therefore tyhe product in position orto- is energetically unfavoured. The solvent and the nitrating mix are recovered and disposed of by conventional methods.

The third reaction is a catalytic reduction with hydrogen of the reaction product of the second reaction, i.e. of the nitration of neopentyl phenyl ether.

The fourth reaction is an acetylation, in position para-, of the reaction product of the third reaction, of catalytic reduction. The fourth reaction may be carried out with acetyl chloride in anhydrous pyridine, because oxygen, blocked in ethereal form, cannot be acetylated.

As the byproduct of the fourth reaction only pyridinium chloride is formed, which can be titrated with soda and thereafter, once sodium chloride removed, pyridine can be recovered by azeotropic distillation.

The fifth reaction is the final one, which brings to the formation of pure paracetamol. It is a simple cleavage of an ether with hydrobromic acid in pentane, benzene or another solvent. Particularly a solvent that is cheap and has minimum environmental impact and disposal costs may be selected for the purpose. Water not being present in the reaction environment one does not have the hydrolysis of the amide.

In this reaction pure paracetamol and neopentyl bromide is formed. As this one constitutes the reactant of use in the first reaction, it may be returned to the first reaction.

In the embodiment referred above of the use of sodium phenoxide, the cleavage of the ether is carried out with hydriodic acid, with formation of neopentyl iodide, which can be returned to the first reaction.

The present invention has been disclosed referring to preferred embodiments thereof, but variations, additions or omissions can be made without departing from the scope of protection relevant thereto, which only remains defined by the enclosed Claims.

### INDUSTRIAL APPLICABILITY

The disclosed method is suitable for producing paracetamol industrially.

## Claims

1. Method for producing paracetamol including the stages of:
*(a)* reacting phenol and neopentyl bromide or sodium phenoxide and neopentyl iodide in a solvent, with formation of neopentylphenyl ether;
*(b)* nitrating said neopentylphenyl ether with formation of a nitro compound of neopentylphenyl ether with an NO₂-group in position para-;
*(c)* catalytically reducing said nitro compound with hydrogen with formation of a phenyl amine;
*(d)* acetylating said phenyl amine in position para-;
*(e)* cleaving said neopentylphenyl ether with hydrobromic acid in an inert solvent or respectively with hydriodic acid in an inert solvent, with formation of paracetamol and neopentyl bromide or respectively neopentyl iodide.

2. Method for producing paracetamol according to Claim 1, further including the stage of:
*(f)* recovering said paracetamol by filtration, and recovering said neopentyl bromide or respectively neopentyl iodide by distillation and returning it to stage *(a)*.

## Patentansprüche

1. Verfahren zum Herstellen von Paracetamol umfaßend die Stufen von:
*(a)* Umsetzen von Phenols und Neopentylbromides beziehungsweise Natriumphenoxides und Neopentyljodides in einem Lösungsmittel, unter Bildung von Neopentylphenyläthers;
*(b)* Nitrieren gesagten Neopentylphenyläthers unter Bildung einer Nitroverbindung von Neopentylphenyläthers mit einer NO₂-Gruppe in para-Position;
*(c)* katalytisch Reduzieren gesagter Nitroverbindung mit Wasserstoff unter Bildung eines Phenylamines;
*(d)* Acetylieren gesagten phenylamines in para-Position;
*(e)* Spalten gesagten Neopentylphenyläthers mit Bromwasserstoffsäure in einem inerten Lösungs-mittel beziehungsweise mit Iodwasserstoffsaüre in einem inerten Lösungsmittel, unter Bildung von Paracetamol und Neopentylbromides beziehungsweise Neopentyljodides.

2. Verfahren zum Herstellen von Paracetamol nach Anspruch 1, ferner umfaßend die Stufe von:
*(f)* Zurückgewinnen gesagten paracetamols durch Filtrierung, und das Zurückgewinnen gesagten Neo-pentylbromides beziehungsweise Neopentyljodides durch Destillation und das Zurückkommen davon zu der Stufe *(a)*.

## Revendications

1. Procédé de production de paracétamol comprenant les étages de:
*(a)* faire réagir du phénol et du bromure de néopentyle ou du phénoxyde de sodium et du iodure de néopentyle dans un solvant, avec formation d'éther néopentylphénylique;
*(b)* nitrer ledit éther néopentylphénylique avec formation d'un nitrocomposé d'éther néopentylphénylique avec un groupe NO₂ en position para- ;
*(c)* réduire catalytiquement ledit nitrocomposé avec de l'hydrogène avec formation d'une phénylamine;
*(d)* acétyler ladite phénylamine en position para-;
*(e)* cliver ledit éther néopentylphénylique avec de l'acide bromhydrique dans un solvant inerte ou respectivement avec de l'acide iodhydrique dans un solvant inerte, avec formation de paracétamol et bromure de néopentyle ou respectivement iodure de néopentyle.

2. Procédé de production de paracétamol selon la Revendication 1, ultérieurement comprenant l'étage de :
*(f)* récupérer ledit paracétamol par filtration, et récupérer ledit bromure de néopentyle ou respectivement iodure de néopentyle par distillation et le faire retourner à l'étage *(a)*.
